(19) Europäisches Patentamt
European Patent Office
Office européen des brevets

(11) **EP 4 279 580 B1**

(12) **EUROPEAN PATENT SPECIFICATION**

(45) Date of publication and mention
of the grant of the patent:
**11.03.2026 Bulletin 2026/11**

(21) Application number: **22778607.6**

(22) Date of filing: **18.03.2022**

(51) International Patent Classification (IPC):
*C12N 5/04* (2006.01)    *A01H 4/00* (2006.01)

(52) Cooperative Patent Classification (CPC):
**A01H 4/00; A01H 4/002; A01H 5/06; A01H 6/00**

(86) International application number:
**PCT/CN2022/081651**

(87) International publication number:
**WO 2022/206424 (06.10.2022 Gazette 2022/40)**

(54) **METHOD FOR SEPARATING AND CULTURING GINSENG STEM CELLS**

VERFAHREN ZUR TRENNUNG UND KULTIVIERUNG VON GINSENG-STAMMZELLEN

PROCÉDÉ DE SÉPARATION ET DE CULTURE DE CELLULES SOUCHES DE GINSENG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.04.2021 CN 202110361871**

(43) Date of publication of application:
**22.11.2023 Bulletin 2023/47**

(73) Proprietor: **Shanghai Kodilin Technology Co., Ltd Shanghai 201800 (CN)**

(72) Inventors:
• **GAO, Xiujun**
**Weihai, Shandong 264205 (CN)**
• **LIU, Bing**
**Weihai, Shandong 264205 (CN)**
• **ZHANG, Mingchen**
**Weihai, Shandong 264205 (CN)**
• **YAN, Peisheng**
**Weihai, Shandong 264205 (CN)**

(74) Representative: **ABG Intellectual Property Law, S.L.**
**Avenida de Burgos, 16D**
**Edificio Euromor**
**28036 Madrid (ES)**

(56) References cited:
EP-A1- 2 962 552          CN-A- 103 509 749
CN-A- 104 472 359      CN-A- 111 876 368
CN-A- 113 025 552      CN-A- 113 046 297
CN-A- 113 151 144      CN-A- 113 174 360

• EUN-KYONG LEE ET AL: "Cultured cambial meristematic cells as a source of plant natural products", NATURE BIOTECHNOLOGY, vol. 28, no. 11, 1 November 2010 (2010-11-01), pages 1213 - 1217, XP055029563, ISSN: 1087-0156, DOI: 10.1038/nbt.1693
• BAOBAO QIANG ET AL: "Recent Advances in the Tissue Culture of American Ginseng (Panax quinquefolius)", CHEMISTRY & BIODIVERSITY, HELVETICA CHIMICA ACTA, ZUERICH, CH, vol. 17, no. 10, 14 October 2020 (2020-10-14), pages n/a, XP072357535, ISSN: 1612-1872, DOI: 10.1002/CBDV.202000366
• SIVAKUMAR G ET AL: "Production of Biomass and Ginsenosides from Adventitious Roots ofPanax ginseng in Bioreactor Cultures", ENGINEERING IN LIFE SCIENCES, vol. 5, no. 4, 1 August 2005 (2005-08-01), DE, pages 333 - 342, XP093173044, ISSN: 1618-0240, DOI: 10.1002/elsc.200520085
• XU CHUN-MING, YING-YING WANG, PANG GAO-YANG, DAN LI: "Stem cell culture technique of medicinal plants and its application", CHINESE TRADITIONAL AND HERBAL DRUGS, TAINJIN ZHONGCAOYAO ZAZAHISHE, CN, vol. 44, no. 20, 28 October 2013 (2013-10-28), CN, pages 2940 - 2945, XP055972130, ISSN: 0253-2670, DOI: 10.7501/j.issn.0253-2670.2013.20.026

EP 4 279 580 B1

- LEE, E. ET AL.: "Cultured cambial meristematic cells as a source of plant natural products", NATURE BIOTECHNOLOGY, vol. 28, no. 11, 24 October 2010 (2010-10-24), XP055029563, ISSN: 1087-0156, DOI: 10.1038/nbt.1693

## Description

### Field

The present invention relates to the technical field of plant tissue culture, and in particular relates to a method for separating and culturing ginseng stem cells.

### Background

Ginseng (Panax ginseng C. A. Mey.) is a plant of the genus Panax of Araliaceae, distributed in China, Japan, and Korea, and its rhizome is a precious Chinese medicinal material, known as the "king of herbs". Ginseng tastes sweet and slightly bitter, and is slightly warm. It has the effects of reinforcing vital energy, restoring the pulse for relieving desertion, tonifying the spleen and benefiting the lung, promoting fluid production and nourishing blood, calming the nerves and improving the intelligence. It is mainly used for verging on desertion due to weak health, reducing appetite due to spleen deficiency, asthma and cough due to lung deficiency, thirst due to body fluid deficiency, insomnia due to palpitation, etc. Ginsenoside is the main active ingredient of ginseng, and has the effects of resisting fatigue, delaying aging, regulating the central nervous system, improving the immunity of the body, improving the blood supply insufficiency of heart and cerebral vessels, and inhibiting the production of tumor cells. In recent years, ginseng has been widely used in various cosmetics, health care products and drinks, and the market prospect of ginseng is very broad.

At present, wild ginseng resources are almost extinct due to excessive digging, environmental destruction, etc., and field cultivation is the main source of ginseng. However, ginseng grows slowly has a long planting period, and requires strict environmental conditions. Its quality is easily affected by climate, cultivation conditions, diseases and insect pests. Its cultivation techniques are complex, and there are also problems such as excessive pesticide residues and old ginseng soil. These problems greatly limit the development prospects of artificial cultivation of ginseng. The supply of ginseng cultivated in the field is difficult to meet the market demand. Ginseng tissue culture technology has a short cycle, is not limited by seasons, and is prone to large-scale industrial production, which has a great development prospect.

At present, adventitious roots are generally induced by ginseng callus, which needs to induce callus first and then induce adventitious roots, which requires a long experimental cycle, and has complex operation steps and high pollution risk. In addition, there is also a problem that the content of ginsenoside in cultivated ginseng is low, which is difficult to meet the needs of clinical applications. For example, CN104472359A discloses a method for inducing the proliferation of ginseng adventitious roots, which comprises the following steps: cutting ginseng tissue culture seedlings into small tissue pieces, inoculating the small tissue pieces into a solid induction medium to induce formation of adventitious roots, cutting the adventitious roots into small adventitious root pieces, and inoculating the small adventitious root pieces into a liquid proliferation medium for proliferation culture of adventitious roots; wherein the solid induction medium and the liquid proliferation medium use a 1/2 MS (-N) medium as a minimal medium, and contains 1-10 mg/L of indolebutyric acid. In CN104472359A, the tissue culture seedlings of 28-32 days old are cut into small pieces to directly induce adventitious roots, the tissue culture seedlings are young and tender, and have strong differentiation ability. In fact, it takes at least 28-32 days to obtain tissue culture seedlings through seed germination or explant culture, and explant culture still needs callus induction. Thus, the cycle is not actually shortened.

Eun-Kyong Lee et al. (Nature Biotechnology (2010) 28, 1213-1217) discloses cultured cambial meristematic cells as a source of plant natural products. EP2962552A1 discloses recombinant plant cell, preparation method therefor, and method for producing target protein using same. Baobao Qiang et al. (Chem. Biodiversity (2020) 17) discloses recent advances in the tissue culture of American ginseng (panax quinquefolius). Sivakumar G et al. (Eng. Life Sci. (2005) 5: 333-342) discloses production of biomass and ginsenosides from adventitious roots of panax ginseng in bioreactor cultures.

Ginseng stem cells are undifferentiated cells with infinite division capacity. At present, ginseng related pharmaceutical active ingredients can be produced by isolating and culturing ginseng stem cells. At present, in the prior art, when ginseng stem cells are isolated, differentiated tissues and organs are used as explants, and are induced into callus cells with differentiation capacity through culture in a three-dimensional culture stage (a dedifferentiation process). But the cells are intrinsically derived from differentiated somatic cells, and therefore have limited division capacity, and low stress resistance. In industrial production, cell line degeneration is prone to occur, and the division capacity is weak.

### Summary

The technical problem to be solved by the present disclosure is to overcome the deficiencies in the prior art and provide a method for separating and culturing ginseng stem cells.

In order to solve the above technical problem, the present invention provides a method for separating and culturing ginseng stem cells as defined in claim 1. Advantageous embodiments are set out in the dependent claims.

The method of the present invention comprises the following steps as defined in claim 1:

(1) culturing ginseng adventitious roots of ginseng by a one-step method;

(2) taking root tips of the ginseng adventitious roots, and dissecting and separating to obtain a stem cell area;

(3) isolation and culture: inoculating the stem cell area into a stem cell induction medium for dark culture to obtain stem cell masses;

(4) subculture: picking part of the stem cell masses obtained in (3) to be inoculated into a stem cell subculture medium for dark culture; and

(5) liquid culture: inoculating stem cells obtained in (4) into a stem cell liquid medium for dark culture to obtain ginseng stem cells.

The root tips of the ginseng adventitious roots have more meristems. According to the method for separating and culturing ginseng stem cells of the present invention, the meristems can be directly dissected and isolated from the root tips of the ginseng adventitious roots without screening in an osmotic environment, and an osmotic agent, which can save steps, shorten the time, and save the cost.

The isolated ginseng stem cells of the present invention are subsequently subjected to dark culture in the stem cell induction medium, the stem cell subculture medium, and the stem cell liquid medium with suitable composition and ratios. The growth speed of ginseng stem cells is fast, and ginseng stem cells can be rapidly obtained.

The present invention uses the one-step method for culturing the ginseng adventitious roots from mature ginseng without an intermediate step of callus induction, and the adventitious roots can be directly induced from the mature ginseng in one step, thereby simplifying the induction steps and shortening the induction time.

In the present invention, in the step (3), the stem cell induction medium includes 2-4 mg/L of gibberellin, 0.6-1 mg/L of kinetin, 2-4 mg/L of indoleacetic acid, 15-75 mg/L of ascorbic acid, 50-150 mg/L of citric acid, 20-60 g/L of sucrose, 1-6 g/L of Phytagel, 1-2.4 g/L of a 1/2 MS medium, and 1-2.5 g/L of a B5 medium.

As one preferred embodiment, the stem cell induction medium includes 3 mg/L of gibberellin, 0.8 mg/L of kinetin, 2.5 mg/L of indoleacetic acid, 50 mg/L of ascorbic acid, 100 mg/L of citric acid, 40 g/L of sucrose, 3 g/L of Phytagel, 1.8 g/L of the 1/2 MS medium, and 1.5 g/L of the B5 medium.

The stem cell induction medium of the present invention contains indoleacetic acid, gibberellin and kinetin, which can induce stem cell growth. And ascorbic acid and citric acid contained in the stem cell induction medium of the present invention can produce synergistic antioxidant effects. The ingredients of the induction medium act synergistically to enable stem cells to rapidly grow in the initial stage.

In the present invention, in the step (4), the stem cell subculture medium includes 2-4 mg/L of 2,4-dichlorophenoxyacetic acid, 1-3 mg/L of gibberellin, 0.8-1.2 mg/L of kinetin, 20-60 g/L of sucrose, 1-6 g/L of Phytagel, 1-2.4 g/L of a 1/2 MS medium, and 0.6-1.4 g/L of a WPM medium.

As one preferred embodiment, the stem cell subculture medium includes 3 mg/L of 2,4-dichlorophenoxyacetic acid, 2 mg/L of gibberellin, 1 mg/L of kinetin, 35 g/L of sucrose, 3 g/L of Phytagel, 1.8 g/L of the 1/2 MS medium, and 0.8 g/L of the WPM medium.

The stem cell subculture medium of the present invention contains 2,4-Dichlorophenoxyacetic acid, gibberellin and kinetin, greatly increasing the growth rate of stem cells.

In a further solution, in the steps (3) and (4), the dark culture is performed at 20-25°C until the inoculated stem cells grow a large number of cell masses.

In the present invention, in the step (5), the stem cell liquid medium includes 2-4 mg/L of 2,4-dichlorophenoxyacetic acid, 1-3 mg/L of gibberellin, 0.8-1.2 mg/L of kinetin, 20-60 g/L of sucrose, 1-2.4 g/L of a 1/2 MS medium and 0.6-1.4 g/L of a WPM medium.

As one preferred embodiment, the stem cell liquid medium includes 3 mg/L of 2,4-dichlorophenoxyacetic acid, 2 mg/L of gibberellin, 1 mg/L of kinetin, 35 g/L of sucrose, 1.8 g/L of the 1/2 MS medium, and 0.8 g/L of the WPM medium.

Further, in the step (5), the dark culture is performed at 20-25°C at 100-150 rpm for passage once every 3-5 weeks.

In a further solution, the pH of the stem cell induction medium, the stem cell subculture medium and the stem cell liquid medium of the present invention is 5.6-6.0.

In a further solution, the pH of the stem cell induction medium, the pH of the stem cell subculture medium, and the pH of the stem cell liquid medium of the present invention are adjusted by using NaOH or KOH to facilitate stem cell growth.

In a further solution, in the step (2), the root tips of the ginseng adventitious roots cultured in (1) are taken and observed under a microscope, the stem cell area is determined based on the meristem cell aggregation area, and a root tip stem cell area is obtained by micromanipulation of dissecting and cutting.

The root tips are observed under the microscope, and irregular parts of cell shape and arrangement are growing points, i.e., a meristem cell aggregation area, which contains multiple vacuoles in the cells. Micromanipulation using the scalpel for cutting is performed to obtain the root tip stem cell area.

In the present invention, in the step (1), a method for culturing the ginseng adventitious roots by the one-step method according to the present invention specifically includes:
washing and disinfecting mature ginseng, cutting the disinfected ginseng into slices, and inoculating the slices into an induction medium to induce adventitious roots of ginseng; re-inoculating the obtained adventitious roots of ginseng into an induction medium for subculture and propagation; and then cutting the obtained adventitious roots of ginseng into segments, and inoculating the segments of the adventitious root of ginseng into a liquid medium for culture to obtain adventitious roots.

At present, adventitious roots are generally induced by ginseng callus, which needs to induce callus first and then induce adventitious roots, which requires a long experimental cycle, and has complex operation steps and high pollution risk. In addition, there is also a problem that the content of ginsenoside in cultivated ginseng is low, which is difficult to meet the needs of clinical applications.

In view of the long age, high maturity and difficulty in differentiation of mature ginseng, there is no report that adventitious roots can be directly induced from mature ginseng. After a large number of experiments, the present invention unexpectedly found that adventitious roots can be directly induced from mature ginseng slices in a specific induction medium, so that the adventitious roots can be directly induced from the mature ginseng in one step without an intermediate step of callus induction, thereby simplifying the induction steps and shortening the induction time.

In the present invention, in the step (1), the induction medium includes 1-6 mg/L of naphthylacetic acid, 0.2-1 mg/L of gibberellin, 0.1-0.6 mg/L of kinetin, 0.075-1.5 g/L of citric acid, 0.03-1 g/L of ascorbic acid, 20-60 g/L of sucrose, 1-6 g/L of Phytagel, 1-4 g/L of a B5 medium, and 1-2.4 g/L of a WPM medium.

This induction medium enables direct induction of adventitious roots from the parts of the mature ginseng without the intermediate step of callus induction, thereby simplifying the induction steps and shortening the induction time.

In this induction medium, naphthylacetic acid is a plant growth regulator and gibberellin is a plant hormone, which can both promote adventitious root formation. Kinetin is a cytokinin that can promote division of cells. Citric acid and ascorbic acid can produce synergistic antioxidant effects, prevent browning of mature ginseng tissue in vitro, and facilitate direct induction of adventitious roots from the mature ginseng tissue in vitro. The ingredients in the induction medium act synergistically, eventually achieving direct induction of adventitious roots from the parts of the mature ginseng without the intermediate step of callus induction.

In a further solution, the induction medium includes 4 mg/L of naphthylacetic acid, 0.6 mg/L of gibberellin, 0.4 mg/L of kinetin, 0.1 g/L of citric acid, 0.05 g/L of ascorbic acid, 30 g/L of sucrose, 3 g/L of Phytagel, 1.55 g/L of a B5 medium and 1.21 g/L of a WPM medium.

The induction medium in the above ratio of ingredients has the best effect on the induction of adventitious roots from mature ginseng. The number of the produced adventitious roots is large and the quality is good, which is conducive to next propagation, and increases the content of active ingredients in adventitious roots.

In a further solution, the liquid medium uses a B5 medium, a WPM medium or a 1/2 MS medium as a basic medium, and contains 4 mg/L of indolebutyric acid, 0.1 g/L of citric acid, 0.05 g/L of ascorbic acid, 30 g/L of sucrose.

In the present invention, when further liquid culture is carried out on the induced adventitious roots, a conventional medium, such as a 1/2 MS medium, can be adopted, which is consistent with the commonly used adventitious root medium, indicating that the adventitious roots induced by the one-step method of the present invention, and adventitious roots obtained by other two-step processes have no difference, and both can be cultured by using the conventional medium, and meanwhile, the induction time is shortened and the pollution risk is reduced.

In a further solution, the slices are thin slices having a width of 0.5-0.7 cm, a length of 0.5-0.7 cm, and a thickness of 0.2-0.5 mm.

In a further solution, the mature ginseng has an age of 3 years or more;

preferably, the mature ginseng has an age of 6 years or more; and
as one preferred embodiment, the mature ginseng is centennial ginseng.

The centennial ginseng is very rare in nature, and has a high edible and medicinal value, and can nourish the five internal organs, soothe the nerves, calm the soul, stop palpitation, eliminate evil spirits, improve eyesight, and make happy and improve intelligence. And its value is much greater than that of planted ginseng with a short age. The present invention does not require the intermediate step of callus induction, adventitious roots can be directly induced from centennial ginseng slices in one step, which can not only simplify the induction steps, and shorten the induction time, but also obtain unique functional components in the female parent centennial ginseng, thereby obtaining adventitious roots with better nutritional values.

In a further solution, taproots, or rhizomes, or adventitious roots on rhizomes, or lateral roots, or fibrous roots of the mature ginseng are washed and disinfected, sliced and inoculated into an induction medium to induce ginseng adventitious roots.

In a further solution, the ginseng is selected from wild ginseng, transplant wild ginseng, ginseng under forest, and garden ginseng; and
preferably, the ginseng is wild ginseng.

As one specific preferred embodiment, in the step (1), culturing the adventitious roots by the one-step method specifically includes the following steps:

(1) induction of adventitious roots
taproots of mature ginseng are washed and sterilized, cut into slices having a width of 0.5-0.7 cm, a length of 0.5-0.7 cm, and a thickness of 0.2-0.5 mm, and inoculated into an induction medium containing 4 mg/L of naphthylacetic acid, 0.6 mg/L of gibberellin, 0.4 mg/L of kinetin, 0.1 g/L of citric acid, 0.05 g/L of ascorbic acid, 30 g/L of sucrose, 3 g/L of Phytagel, 1.55 g/L of a B5 medium and 1.21 g/L of a WPM medium for dark culture at 22±1°C for 4-5 weeks to induce ginseng adventitious roots;

(2) subculture of adventitious roots
the ginseng adventitious roots obtained in the step (1) are inoculated into the same induction medium as that in the step (1) for dark culture under the same conditions for 4-5 weeks; and

(3) culture of adventitious roots
the ginseng adventitious roots obtained in the step (2) are cut into tissue of about 1-2 cm in length, and inoculated into a liquid medium containing 4 mg/L of indolebutyric acid, 0.1 g/L of citric acid, 0.05 g/L of ascorbic acid, a 1/2 MS medium, and 30 g/L of sucrose for culture on a shaker at 22±1°C for 3-4 weeks to obtain adventitious roots.

In a further solution, the pH of the induction medium or the liquid medium of the present invention is 5.6-6.0.

In a further solution, the pH of the induction medium and the pH of the liquid medium of the present invention are adjusted by using NaOH or KOH to facilitate adventitious root growth.

It should be noted that in the present invention, the WPM medium, the B5 medium, the 1/2 MS medium, and the like are known in the art.

1/2 MS medium

Ingredients are as follows: 950 mg/L of potassium nitrate, 825 mg/L of ammonium nitrate, 220 mg/L of calcium chloride dihydrate, 185 mg/L of magnesium sulfate, 85 mg/L of potassium dihydrogen phosphate, 11.15 mg/L of manganese sulfate, 4.3 mg/L of zinc sulfate, 3.1 mg/L of boric acid, 0.415 mg/L of potassium iodide, 0.125 mg/L of sodium molybdate, 0.0125 mg/L of copper sulfate, 0.0125 mg/L of cobalt chloride, 37.3 mg/L of disodium ethylenediamine tetraacetate, 27.8 mg/L of ferrous sulfate, 100 mg/L of inositol, 2 mg/L of glycine, 0.5 mg/L of hydrochloric acid, 0.5 mg/L of pyridoxine hydrochloride, and 0.1 mg/L of thiamine hydrochloride.

B5 medium

Ingredients are as follows: 2500 mg/L of potassium nitrate $KNO_3$, 250 mg/L of $MgSO_4 \cdot 7H_2O$, 150 mg/L of $CaCl_2 \cdot 2H_2O$, 134 mg/L of $(NH_4)_2SO_4$, 150 mg/L of $NaH_2PO_4 \cdot H_2O$, 0.075 mg/L of KI, 3.0 mg/L of $H_3BO_3$, 10 mg/L of $MnSO_4 \cdot 4H_2O$, 2.0 mg/L of $ZnSO_4 \cdot 7H_2O$, 0.25 mg/L of $Na_2MoO_4 \cdot 2H_2O$, 0.025 mg/L of $CoCl_2 \cdot 6H_2O$, 0.025 mg/L of $CuSO_4 \cdot 5H_2O$, 37.3 mg/L of $Na_2$-EDTA, 27.8 mg/L of $FeSO_4 \cdot 7H_2O$, 100 mg/L of inositol, 1.0 mg/L of nicotinic acid, 1.0 mg/L of pyridoxine hydrochloride, and 10 mg/L of thiamine hydrochloride.

Woody plant medium (WPM)

Ingredients are as follows: 400 mg/L of ammonium nitrate, 556 mg/L of calcium nitrate tetrahydrate, 990 mg/L of potassium sulfate, 72 mg/L of anhydrous calcium chloride, 170 mg/L of potassium dihydrogen phosphate, 0.25 mg/L of sodium molybdate dihydrate, 180 mg/L of anhydrous magnesium sulfate, 22.4 mg/L of manganese sulfate monohydrate, 8.6 mg/L of zinc sulfate heptahydrate, 0.25 mg/L of copper sulfate pentahydrate, 27.8 mg/L of ferrous sulfate heptahydrate, 37.3 mg/L of disodium ethylenediamine tetraacetate, 100 mg/L of inositol, 1 mg/L of vitamin B1, 0.5 mg/L of nicotinic acid, 0.5 mg/L of vitamin B6, and 2 mg/L of glycine, with a pH of 5.2.

After the above technical solution is adopted, compared with the prior art, the present invention has the following beneficial effects:

1. The root tips of the ginseng adventitious roots have more meristems. According to the method for separating and culturing ginseng stem cells of the present invention, the root tips are observed under the microscope, the meristems, that is, the stem cell area, which contains multiple vacuoles in the cells, are directly dissected and isolated from the root tips of the ginseng adventitious roots without screening in an osmotic environment, and an osmotic agent, which can save steps, shorten the time, and save the cost.

2. The isolated ginseng stem cells of the present invention are subsequently subjected to dark culture in the stem cell induction medium, the stem cell subculture medium, and the stem cell liquid medium with suitable composition and ratios, and grow fast, and ginseng stem cells can be rapidly obtained.

3. The ginseng adventitious roots used in the present invention are obtained by one-step culture, and can be directly induced by inoculating the parts of the mature ginseng into the induction medium after treatment without the intermediate step of callus induction, which simplifies the induction steps, shortens the induction time and reduces the pollution risk.

4. In the present invention, the induction medium used in the method for culturing ginseng adventitious roots includes 1-6 mg/L of naphthylacetic acid, 0.2-1 mg/L of gibberellin, 0.1-0.6 mg/L of kinetin, 0.075-1.5 g/L of citric acid, 0.03-1 g/L of ascorbic acid, 20-60 g/L of sucrose, 1-6 g/L of Phytagel, 1-4 g/L of the B5 medium and 1-2.4 g/L of the WPM medium, and the ingredients act synergistically, and the problem of direct induction of the adventitious roots from the mature ginseng by the one-step method is solved, resulting in a large number of adventitious roots.

Specific embodiments of the present discolsure will be further described below in detail in combination with the accompanying drawings.

**Brief Description of the Drawings**

The drawings serving as one part of the present invention are intended to provide a further understanding for the present invention. Schematic embodiments of the present invention and the descriptions thereof are intended to explain the present invention, rather than an improper limitation of the present invention. Obviously, the drawings described below are merely some embodiments. On the premise of not paying inventive labor, those of ordinary skill in the art can further obtain other drawings according to these drawings. In the drawings:

Fig. 1 is a schematic diagram of induction of adventitious roots by using an induction medium in Embodiment 1 of the present invention;
Fig. 2 is a cell image of stem cells cultured in Embodiment 7 of the present invention after being magnified 1000 times under a microscope; and
Fig. 3 is a schematic diagram of induction of adventitious roots by using an induction medium in Comparative example 1.

**Detailed Description of the Embodiments**

To make the objectives, technical solutions, and advantages of the embodiments of the present invention clearer, the technical solutions in the embodiments will be clearly and completely described below. The following embodiments are used to describe the present invention but not to limit the scope of the present invention.

It should be noted that a preparation method or a detection method, which is not specifically defined in the present invention, can be performed by using a method that can be used to achieve its purpose in the prior art of the field.

Embodiment 1

(1) Induction of adventitious roots
Rhizomes and adventitious roots on rhizomes were removed from 20-year-old wild ginseng, leaving the taproots. The taproots were washed and sterilized, cut into slices having a width of 0.6 cm, a length of 0.7 cm, and a thickness of 0.3 mm, and inoculated into an induction medium for dark culture at 22±1°C for 4-5 weeks to induce wild ginseng adventitious roots; wherein the induction medium includes 4 mg/L of naphthylacetic acid, 0.6 mg/L of gibberellin, 0.4 mg/L of kinetin, 0.1 g/L of citric acid, 0.05 g/L of ascorbic acid, 30 g/L of sucrose, 3 g/L of Phytagel, 1.55 g/L of a B5 medium and 1.21 g/L of a WPM medium, with a pH of 5.8;
(2) subculture of adventitious roots
the wild ginseng adventitious roots obtained in the step (1) were inoculated into the same induction medium as that in the step (1) for dark culture under the same conditions for 4-5 weeks; and
(3) culture of adventitious roots
the wild ginseng adventitious roots obtained in the step (2) were cut into tissue of about 1 cm in length, and inoculated into a liquid medium for culture on a shaker at 22±1°C at 130 rpm for 3-4 weeks to obtain adventitious roots; wherein the liquid medium contains 4 mg/L of indolebutyric acid, 0.1 g/L of citric acid, 0.05 g/L of ascorbic acid, a 1/2 MS medium and 30 g/L of sucrose, with a pH of 5.8.

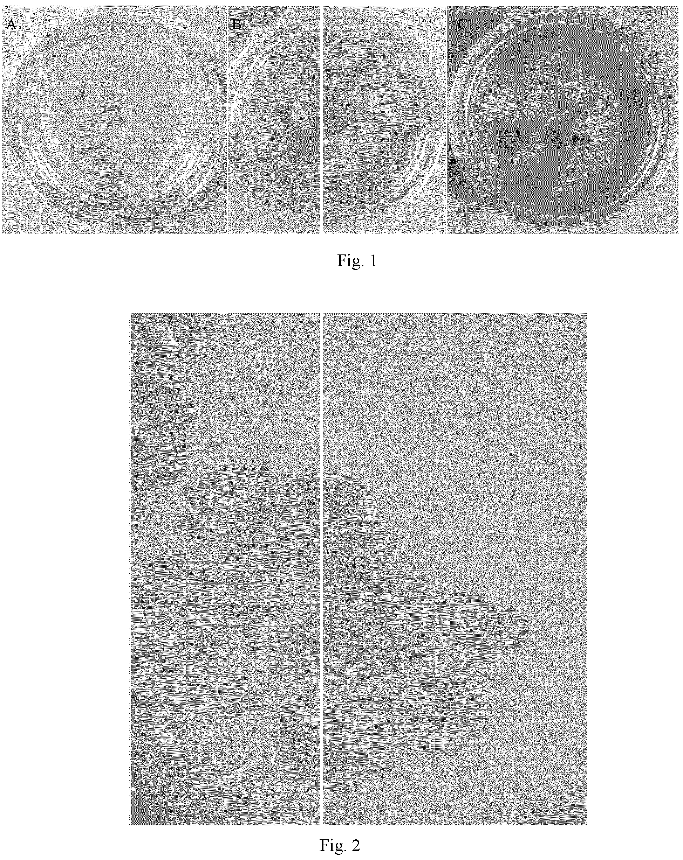

In this embodiment, the adventitious roots produced on the induction medium in the step (1) are shown in Fig. 1, wherein A is a photograph of 1 week of culture, B is a photograph of 3 weeks of culture, and C is a photograph of 5 weeks of culture. It can be seen that after 5 weeks, adventitious roots were directly induced on the mature wild ginseng slices.

Embodiment 2

(1) Induction of adventitious roots

Rhizomes and adventitious roots on rhizomes of 10-year-old wild ginseng were removed, leaving the taproots. The taproots were washed and sterilized, cut into slices having a width of 0.5 cm, a length of 0.6 cm, and a thickness of 0.4 mm, and inoculated into an induction medium for dark culture at 22±1°C for 4-5 weeks to induce wild ginseng adventitious roots; wherein the induction medium includes 4 mg/L of naphthylacetic acid, 0.6 mg/L of gibberellin, 0.4 mg/L of kinetin, 0.1 g/L of citric acid, 0.05 g/L of ascorbic acid, 30 g/L of sucrose, 3 g/L of Phytagel, 1.55 g/L of a B5 medium and 1.21 g/L of a WPM medium, with a pH of 5.8;

(2) subculture of adventitious roots

the wild ginseng adventitious roots obtained in the step (1) were inoculated into the same induction medium as that in the step (1) for dark culture under the same conditions for 4-5 weeks; and

(3) culture of adventitious roots

the wild ginseng adventitious roots obtained in the step (2) were cut into tissue of about 1.5 cm in length, and inoculated into a liquid medium for culture on a shaker at 22±1°C at 120 rpm for 3-4 weeks to obtain adventitious roots; wherein the liquid medium contains 4 mg/L of indolebutyric acid, 0.1 g/L of citric acid, 0.05 g/L of ascorbic acid, a 1/2 MS medium and 30 g/L of sucrose, with a pH of 5.8.

Similar to the results of Embodiment 1, adventitious roots can be induced in one step in the step (1) of this embodiment.

Embodiment 3

(1) Induction of adventitious roots

Rhizomes of 6-year-old garden ginseng were washed and sterilized, cut into slices having a width of 0.5 cm, a length of 0.6 cm, and a thickness of 0.3 mm, and inoculated into an induction medium for dark culture at 22±1°C for 4-5 weeks to induce adventitious roots; wherein the induction medium includes 6 mg/L of naphthylacetic acid, 0.2 mg/L of gibberellin, 0.4 mg/L of kinetin, 1.2 g/L of citric acid, 0.1 g/L of ascorbic acid, 20 g/L of sucrose, 5 g/L of Phytagel, 4 g/L of a B5 medium and 1.8 g/L of a WPM medium, with a pH of 5.6;

(2) subculture of adventitious roots

the adventitious roots obtained in the step (1) were inoculated into the same induction medium as that in the step (1) for dark culture under the same conditions for 4-5 weeks; and

(3) culture of adventitious roots

the adventitious roots obtained in the step (2) were cut into tissue of about 1 cm in length, and inoculated into a liquid medium for culture on a shaker at 22±1°C at 110 rpm for 3-4 weeks to obtain adventitious roots; wherein the liquid medium contains 4 mg/L of indolebutyric acid, 0.1 g/L of citric acid, 0.05 g/L of ascorbic acid, a 1/2 MS medium and 30 g/L of sucrose, with a pH of 5.6.

Similar to the results of Embodiment 1, adventitious roots can be induced in one step in the step (1) of this embodiment.

Embodiment 4

(1) Induction of adventitious roots

Adventitious roots on rhizomes of 15-year-old ginseng under forest were washed and sterilized, cut into slices having a width of 0.7 cm, a length of 0.7 cm, and a thickness of 0.5 mm, and inoculated into an induction medium for dark culture at 22±1°C for 4-5 weeks to induce adventitious roots; wherein the induction medium includes 5 mg/L of naphthylacetic acid, 1 mg/L of gibberellin, 0.1 mg/L of kinetin, 0.075 g/L of citric acid, 0.03 g/L of ascorbic acid, 40 g/L of sucrose, 4 g/L of Phytagel, 2 g/L of a B5 medium and 1 g/L of a WPM medium, with a pH of 6.0;
(2) subculture of adventitious roots
the adventitious roots obtained in the step (1) were inoculated into the same induction medium as that in the step (1) for dark culture under the same conditions for 4-5 weeks; and
(3) culture of adventitious roots
the adventitious roots obtained in the step (2) were cut into tissue of about 2 cm in length, and inoculated into a liquid medium for culture on a shaker at 22±1°C at 130 rpm for 3-4 weeks to obtain adventitious roots; wherein the liquid medium contains 4 mg/L of indolebutyric acid, 0.1 g/L of citric acid, 0.05 g/L of ascorbic acid, a WPM medium and 30 g/L of sucrose, with a pH of 6.0.

Similar to the results of Embodiment 1, adventitious roots can be induced in one step in the step (1) of this embodiment.

Embodiment 5

(1) Induction of adventitious roots
Taproots of 20-year-old transplant wild ginseng were washed and sterilized, cut into slices having a width of 0.5 cm, a length of 0.6 cm, and a thickness of 0.4 mm, and inoculated into an induction medium for dark culture at 22±1°C for 4-5 weeks to induce wild ginseng adventitious roots; wherein the induction medium includes 1 mg/L of naphthylacetic acid, 0.5 mg/L of gibberellin, 0.6 mg/L of kinetin, 1.5 g/L of citric acid, 1 g/L of ascorbic acid, 50 g/L of sucrose, 6 g/L of Phytagel, 1 g/L of a B5 medium and 2.4 g/L of a WPM medium, with a pH of 5.7;
(2) subculture of adventitious roots
the wild ginseng adventitious roots obtained in the step (1) were inoculated into the same induction medium as that in the step (1) for dark culture under the same conditions for 4-5 weeks; and
(3) culture of adventitious roots
the wild ginseng adventitious roots obtained in the step (2) were cut into tissue of about 1 cm in length, and inoculated into a liquid medium for culture on a shaker at 22±1°C for 3-4 weeks to obtain adventitious roots; wherein the liquid medium contains 4 mg/L of indolebutyric acid, 0.1 g/L of citric acid, 0.05 g/L of ascorbic acid, a B5 medium and 30 g/L of sucrose, with a pH of 5.7.

Similar to the results of Embodiment 1, adventitious roots can be directly induced in one step in the step (1) of this embodiment.

Embodiment 6

(1) Induction of adventitious roots
Rhizomes and adventitious roots on rhizomes of centennial wild ginseng were removed, leaving the taproots. The taproots were washed and sterilized, cut into slices having a width of 0.6 cm, a length of 0.7 cm, and a thickness of 0.3 mm, and inoculated into an induction medium for dark culture at 22±1°C for 4-5 weeks to induce wild ginseng adventitious roots; wherein the induction medium includes 4 mg/L of naphthylacetic acid, 0.6 mg/L of gibberellin, 0.4 mg/L of kinetin, 0.1 g/L of citric acid, 0.05 g/L of ascorbic acid, 30 g/L of sucrose, 3 g/L of Phytagel, 1.55 g/L of a B5 medium and 1.21 g/L of a WPM medium, with a pH of 5.8;
(2) subculture of adventitious roots
the wild ginseng adventitious roots obtained in the step (1) were inoculated into the same induction medium as that in the step (1) for dark culture under the same conditions for 4-5 weeks; and
(3) culture of adventitious roots
the wild ginseng adventitious roots obtained in the step (2) were cut into tissue of about 1 cm in length, and inoculated into a liquid medium for culture on a shaker at 22±1°C at 130 rpm for 3-4 weeks to obtain adventitious roots; wherein the liquid medium contains 4 mg/L of indolebutyric acid, 0.1 g/L of citric acid, 0.05 g/L of ascorbic acid, a 1/2 MS medium and 30 g/L of sucrose, with a pH of 5.8.

Similar to the results of Embodiment 1, adventitious roots can be directly induced in one step in the step (1) of this embodiment.

Embodiment 7 Culture of ginseng stem cells

(1) the ginseng adventitious roots prepared in Embodiment 2 were used;
(2) isolation of stem cells from root tips of adventitious roots: root tips of ginseng adventitious roots in (1) were taken, and subjected to micromanipulation of dissecting and cutting to obtain a root tip stem cell area;
(3) isolation and culture: the stem cell area was inoculated into a stem cell induction medium for dark culture at 22°C until the inoculated stem cells grow a large number of cell masses; wherein

the stem cell induction medium includes: 3 mg/L of gibberellin, 0.8 mg/L of kinetin, 2.5 mg/L of indoleacetic acid, 50 mg/L of ascorbic acid, 100 mg/L of citric acid, 40 g/L of sucrose, 3 g/L of Phytagel, 1.8 g/L of a 1/2 MS medium, and 1.5 g/L of a B5 medium, with a pH of 5.8;

(4) subculture: part of the stem cell masses obtained in (3) was picked and inoculated into a stem cell subculture medium for dark culture at 22°C until the inoculated stem cells grow a large number of cell masses; wherein

the stem cell subculture medium includes: 3 mg/L of 2,4-dichlorophenoxyacetic acid, 2 mg/L of gibberellin, 1 mg/L of kinetin, 35 g/L of sucrose, 3 g/L of Phytagel, 1.8 g/L of a 1/2 MS medium and 0.8 g/L of a WPM medium, with a pH of 5.8; and

(5) liquid culture: the stem cells obtained in (4) were inoculated into a stem cell liquid medium for dark culture at 22°C at 120 rpm for passage once every 3-5 weeks; wherein

the stem cell liquid medium includes 3 mg/L of 2,4-dichlorophenoxyacetic acid, 2 mg/L of gibberellin, 1 mg/L of kinetin, 35 g/L of sucrose, 1.8 g/L of a 1/2 MS medium, and 0.8 g/L of a WPM medium, with a pH of 5.8.

Embodiment 8 Culture of ginseng stem cells

(1) the ginseng adventitious roots prepared in Embodiment 3 were used;
(2) isolation of stem cells from root tips of adventitious roots: root tips of the ginseng adventitious roots in (1) were taken, and subjected to micromanipulation of dissecting and cutting to obtain a root tip stem cell area; (3) isolation and culture: the stem cell area was inoculated into a stem cell induction medium for dark culture at 25°C until the inoculated stem cells grow a large number of cell masses; wherein

the stem cell induction medium includes: 4 mg/L of gibberellin, 0.6 mg/L of kinetin, 2 mg/L of indoleacetic acid, 75 mg/L of ascorbic acid, 50 mg/L of citric acid, 20 g/L of sucrose, 6 g/L of Phytagel, 2.4 g/L of a 1/2 MS medium, 1 g/L of a B5 medium, with a pH of 5.8;

(4) subculture: part of the stem cell masses obtained in (3) was picked and inoculated into a stem cell subculture medium for dark culture at 25°C until the inoculated stem cells grow a large number of cell masses; wherein

the stem cell subculture medium includes: 2 mg/L of 2,4-dichlorophenoxyacetic acid, 3 mg/L of gibberellin, 1.2 mg/L of kinetin, 60 g/L of sucrose, 1 g/L of Phytagel, 1 g/L of a 1/2 MS medium and 1.4 g/L of a WPM medium, with a pH 5.8; and

(5) liquid culture: the stem cells obtained in (4) were inoculated into a stem cell liquid medium for dark culture at 25°C at 150 rpm for passage once every 2 weeks; wherein

the stem cell liquid medium includes 2 mg/L of 2,4-dichlorophenoxyacetic acid, 3 mg/L of gibberellin, 1.2 mg/L of kinetin, 60 g/L of sucrose, 1 g/L of a 1/2 MS medium and 1.4 g/L of a WPM medium, with a pH of 5.8.

Embodiment 9 Culture of ginseng stem cells

(1) the ginseng adventitious roots prepared in Embodiment 4 were used;
(2) isolation of stem cells from root tips of adventitious roots: root tips of the ginseng adventitious roots in (1) were taken, and subjected to micromanipulation of dissecting and cutting to obtain a root tip stem cell area; (3) isolation and culture: the stem cell area was inoculated into a stem cell induction medium for dark culture at 20°C until the inoculated stem cells grow a large number of cell masses; wherein

the stem cell induction medium includes: 2 mg/L of gibberellin, 1 mg/L of kinetin, 4 mg/L of indoleacetic acid, 15 mg/L of ascorbic acid, 150 mg/L of citric acid, 60 g/L of sucrose, 1 g/L of Phytagel, 1 g/L of a 1/2 MS medium, and 2.5 g/L of a B5 medium, with a pH of 5.8;

(4) subculture: part of the stem cell masses obtained in (3) was picked and inoculated into a stem cell subculture medium for dark culture at 22°C until the inoculated stem cells grow a large number of cell masses; wherein

the stem cell subculture medium includes 4 mg/L of 2,4-dichlorophenoxyacetic acid, 1 mg/L of gibberellin, 0.8 mg/L of kinetin, 20 g/L of sucrose, 6 g/L of Phytagel, 2.4 g/L of a 1/2 MS medium and 0.6 g/L of a WPM medium, with a pH of 5.8; and

(5) liquid culture: the stem cells obtained in (4) were inoculated into a stem cell liquid medium for dark culture at 22°C at 120 rpm for passage once every 2 weeks; wherein

the stem cell liquid medium includes 4 mg/L of 2,4-dichlorophenoxyacetic acid, 1 mg/L of gibberellin, 0.8 mg/L of kinetin, 20 g/L of sucrose, 2.4 g/L of a 1/2 MS medium and 0.6 g/L of a WPM medium, with a pH of 5.8.

Comparative example 1

This comparative example differed from Embodiment 1 in that the induction medium used was different and the other steps were carried out with reference to Embodiment 1. The induction medium in this comparative example includes: 30 g/L of sucrose, 0.5 mg/L of kinetin, 3 mg/L of indolebutyric acid, 1.5 mg/L of 2,4-dichlorophenoxyacetic acid, a 1/2 MS medium, and 3 g/L of Phytagel, with a pH of 5.8.

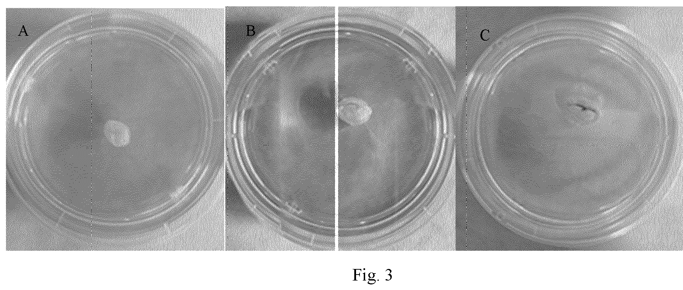

The adventitious roots produced on the induction medium in the step (1) of this comparative example are shown in Fig. 3, wherein A is a photograph of 1 week of culture, B is a photograph of 3 weeks of culture, and C is a photograph of 5 weeks of culture. It can be seen that after 5 weeks, adventitious roots cannot be directly induced from mature wild ginseng slices by using the medium in Comparative example 1.

Comparative example 2

This comparative example differed from Embodiment 1 in that the induction medium used was different and the other steps were carried out with reference to Embodiment 1. The induction medium in this comparative example includes: 30 g/L of sucrose, 0.5 mg/L of kinetin, 3 mg/L of indolebutyric acid, a 1/2 MS medium, and 3 g/L of Phytagel, with a pH of 5.8.

As a result, similar to that shown in the picture in Comparative example 1, adventitious roots cannot be directly induced from mature wild ginseng slices.

Comparative example 3

This comparative example differed from Embodiment 1 in that the induction medium used was different and the other steps were carried out with reference to Embodiment 1. The induction medium in this comparative example includes: 30 g/L of sucrose, 0.5 mg/L of kinetin, 3 mg/L of indoleacetic acid, WPM, and 3 g/L Phytagel, with a pH of 5.8.

Results: in the first week, the whole body turned yellow, in the third week, the color deepened, and the middle part began to turn brown, and in the fifth week, all became brown and withered.

Test example 1

In this example, the wild ginseng stem cells prepared in Embodiment 7 and the wild ginseng slices used in Embodiment 2 were subjected to saponin detection and polysaccharide detection, respectively.

1. Saponin detection method

(1) Principle

After pretreatment such as extraction, a sample was separated by a C18 chromatographic column, and detected by a HPLC-UV detector, and the content of ginsenoside components was determined quantitatively by an external standard method.

(2) Reagents

Methanol ($CH_4O$): chromatographically pure, and acetonitrile ($C_6H_{11}N$): chromatographically pure

Standard reagent: ginsenosides Re, Rg1, Ra3, Rb1, Rf, Rb2, Rb3, F3, Rg2, Rd, and F1.

(3) Analysis steps

Preparation of ginseng stem cell sample:

The ginseng stem cell sample obtained in Embodiment 7 was washed for three times with water, ground to a paste in a mortar, sonicated for wall breaking, and lyophilized. The lyophilized sample was ground on a mortar. 50 mg of the ground sample was accurately weighed to be placed in a 10 ml centrifuge tube, a 70% methanol solution was added, and vortex was conducted. Ultrasonic treatment was conducted on an ultrasonic oscillator for 10 min, the above operation was conducted repeatedly twice, and filtration was conducted for later use.

Preparation of wild ginseng sample:

The wild ginseng slices were washed for three times with water, ground to a paste in a mortar, sonicated for wall breaking, and lyophilized. The lyophilized sample was ground on a mortar, 50 mg of the ground sample was accurately weighed to be placed in a 10 ml centrifuge tube, a 70% methanol solution was added, and vortex was conducted. Ultrasonic treatment was conducted on an ultrasonic oscillator for 10 min, the above operation was conducted repeatedly twice, and filtration was conducted for later use.

Standard preparation:

Preparation of stock solution (0.8 mg/ml): 8.00 mg of ginsenosides Re, Rb1, Rg1, Rd, Rf, F3, Rk2, Rb2, Rb3, Rg2, and F1, which are 11 standards in total, were respectively weighed to be placed in a 10 ml volumetric flask, and the volume was made up to a constant volume with superior pure methanol.

Preparation of use solution (32 $\mu$g/ml): 1 ml of the stock solution (0.8 mg/ml) was accurately pipetted into a 25 ml volumetric flask, and made up to a constant volume with superior pure methanol, and filtered through a 0.22 $\mu$m organic filter membrane for later use.

(4) Instrument reference conditions

A) Chromatographic column: a C18 column with a column length of 150 mm, a column internal diameter of 4.6 mm, and a column packing particle size of 5 $\mu$m, or equivalent;
B) Mobile phase: a: acetonitrile, and b: water filtered through a 0.45 $\mu$m microporous filter membrane;
C) Flow rate: 0.7 mL/min; gradient elution procedure: 0-13 min, 23-46% acetonitrile, with a volume flow rate of 0.7 mL/min; 13-33 min, 46-68% acetonitrile, with a volume flow rate of 0.7 mL/min; 33-46.5 min, 68-85% acetonitrile, with a volume flow rate of 0.7 mL/min;
D) Column temperature: 30°C;
E) Detection wavelength: 203 nm; and
F) Injection volume: 10 $\mu$L.

(5) Expression of analysis results

The content of ginsenoside components in the sample is calculated according to a formula (1):

$$X = \frac{A1}{A2} \times \frac{\rho \times V}{M} \quad (1)$$

in the formula:

X-the content of ginsenoside components in a sample in milligrams per kilogram (mg/kg) or milligrams per liter (mg/L);
A1-peak area of ginsenoside components in a sample
A2-peak area of ginsenoside components in a standard
$\rho$-concentration of ginsenoside components in a standard ($\mu$g/ml)
V-final constant volume of a sample solution in milliliters (mL);
M-sample mass in grams (g);

The content of ginsenoside in the sample is the sum of those detected in the components.

2. Polysaccharide detection method

Detection was performed with reference to a method of the Jilin Province local standard: DB 22/T 1685-2012.

3. Results

The results of ginsenoside detection and polysaccharide detection are shown in Table 1.

Table 1

| Name | Ginsenoside content (mg/kg) | Polysaccharide content (mg/kg) |
|---|---|---|
| Wild ginseng stem cell | 6033.5 | 6485.3 |
| Wild ginseng slices | 4043.8 | 4277.2 |

As can be seen from the above results, both the total content of ginsenosides and the content of polysaccharides were increased in the cultured wild ginseng stem cells of the present invention compared with mature wild ginseng slices. Therefore, the method for separating and culturing ginseng stem cells of the present invention is simple in steps, and time-saving. And the ginseng stem cells grow fast, and have high content of active ingredients.

The above description is only preferred embodiments of the present invention, and is not intended to limit the present invention in any form. Although the present invention has been disclosed above with the preferred embodiments, it is not intended to limit the present invention. Any person familiar with this patent can make some changes or modifications without departing from the scope of the appended claims.

**Claims**

1.    A method for separating and culturing ginseng stem cells, comprising:

(1) culturing adventitious roots of ginseng by a one-step method, the one-step method for culturing the adventitious roots of ginseng comprises:

washing and disinfecting mature ginseng, cutting the disinfected mature ginseng into slices, and inoculating the slices into an adventitious root induction medium to induce adventitious roots of ginseng; re-inoculating the adventitious roots of ginseng obtained into an adventitious root induction medium for subculture and propagation; and then cutting the adventitious roots of ginseng obtained into segments, and inoculating the segments of the adventitious root of ginseng into a liquid medium for culture to obtain adventitious roots; the adventitious root induction medium comprises 1-6 mg/L of naphthylacetic acid, 0.1-0.6 mg/L of kinetin, 0.2-1 mg/L of gibberellin, 0.075-1.5 g/L of citric acid, 0.03-1 g/L of ascorbic acid, 20-60 g/L of sucrose, 1-6 g/L of Phytagel, 1-4 g/L of a B5 medium, and 1-2.4 g/L of a WPM medium;

(2) taking root tips of the adventitious roots of ginseng, and dissecting and separating to obtain a stem cell area;
(3) isolation and culture: inoculating the stem cell area into a stem cell induction medium for dark culture to obtain stem cell masses;
the stem cell induction medium comprises 2-4 mg/L of gibberellin, 0.6-1 mg/L of kinetin, 2-4 mg/L of indoleacetic acid, 15-75 mg/L of ascorbic acid, 50-150 mg/L of citric acid, 20-60 g/L of sucrose, 1-6 g/L of Phytagel, 1-2.4 g/L of a 1/2 MS medium, and 1-2.5 g/L of a B5 medium;
(4) subculture: picking part of the stem cell masses obtained in step (3) to be inoculated into a stem cell subculture medium for dark culture;
the stem cell subculture medium comprises 2-4 mg/L of 2,4-dichlorophenoxyacetic acid, 1-3 mg/L of gibberellin, 0.8-1.2 mg/L of kinetin, 20-60 g/L of sucrose, 1-6 g/L of Phytagel, 1-2.4 g/L of a 1/2 MS medium, and 0.6-1.4 g/L of a WPM medium; and
(5) liquid culture: inoculating stem cells obtained in step (4) into a stem cell liquid medium for dark culture to obtain ginseng stem cells;
the stem cell liquid medium comprises 2-4 mg/L of 2,4-dichlorophenoxyacetic acid, 1-3 mg/L of gibberellin, 0.8-1.2 mg/L of kinetin, 20-60 g/L of sucrose, 1-2.4 g/L of a 1/2 MS medium, and 0.6-1.4 g/L of a WPM medium.

2.    The method for separating and culturing ginseng stem cells according to claim 1, wherein in the step (3), the stem cell induction medium comprises 3 mg/L of gibberellin, 0.8 mg/L of kinetin, 2.5 mg/L of indoleacetic acid, 50 mg/L of ascorbic acid, 100 mg/L of citric acid, 40 g/L of sucrose, 3 g/L of Phytagel, 1.8 g/L of the 1/2 MS medium, and 1.5 g/L of the B5 medium.

3. The method for separating and culturing ginseng stem cells according to claim 1, wherein in the step (4), the stem cell subculture medium comprises 3 mg/L of 2,4-dichlorophenoxyacetic acid, 2 mg/L of gibberellin, 1 mg/L of kinetin, 35 g/L of sucrose, 3 g/L of Phytagel, 1.8 g/L of the 1/2 MS medium, and 0.8 g/L of the WPM medium.

4. The method for separating and culturing ginseng stem cells according to claim 1, wherein in the steps (3) and (4), the dark culture is performed in a range of 20-25°C until the inoculated stem cells grow a large number of cell masses.

5. The method for separating and culturing ginseng stem cells according to claim 1, wherein in the step (5), the stem cell liquid medium comprises 3 mg/L of 2,4-dichlorophenoxyacetic acid, 2 mg/L of gibberellin, 1 mg/L of kinetin, 35 g/L of sucrose, 1.8 g/L of the 1/2 MS medium, and 0.8 g/L of the WPM medium.

6. The method for separating and culturing ginseng stem cells according to any one of claims from 1 to 5, wherein in the step (5), the dark culture is performed in a range of 20-25°C at 100-150 rpm, and passage was conducted once every 3-5 weeks.

7. The method for separating and culturing ginseng stem cells according to any one of claims from 1 to 6, wherein in the step (2), the root tips of the adventitious roots of ginseng cultured in step (1) are taken and observed under a microscope, the stem cell area is determined based on the meristem cell aggregation area, and a root tip stem cell area is obtained by micromanipulation using a scalpel for cutting.

8. The method for separating and culturing ginseng stem cells according to any one of claims from 1 to 5, wherein the ginseng is selected from wild ginseng, transplant wild ginseng, ginseng under forest, and garden ginseng.

9. The method for separating and culturing ginseng stem cells according to claim 8, wherein the ginseng is wild ginseng.

**Patentansprüche**

1. Ein Verfahren zur Trennung und Kultivierung von Ginseng-Stammzellen, umfassend:

   (1) Kultivieren von Adventivwurzeln von Ginseng durch ein einstufiges Verfahren, wobei das einstufige Verfahren zum Kultivieren der Adventivwurzeln von Ginseng umfasst:

      Waschen und Desinfizieren reifen Ginsengs, Schneiden des desinfizierten reifen Ginsengs in Scheiben und Inokulieren der Scheiben in ein Adventivwurzel-Induktionsmedium, um Adventivwurzeln von Ginseng zu induzieren; erneutes Inokulieren der gewonnenen Adventivwurzeln von Ginseng in ein Adventivwurzel-Induktionsmedium zur Subkultur und Vermehrung; und anschließendes Schneiden der gewonnenen Adventivwurzeln von Ginseng in Segmente und Inokulieren der Segmente der Adventivwurzel von Ginseng in ein flüssiges Medium zur Kultivierung, um Adventivwurzeln zu erhalten;
      das Adventivwurzel-Induktionsmedium umfasst 1 bis 6 mg/L Naphthylessigsäure, 0,1 bis 0,6 mg/L Kinetin, 0,2 bis 1 mg/L Gibberellin, 0,075 bis 1,5 g/L Zitronensäure, 0,03 bis 1 g/L Ascorbinsäure, 20 bis 60 g/L Saccharose, 1 bis 6 g/L Phytagel, 1 bis 4 g/L eines B5-Mediums und 1 bis 2,4 g/L eines WPM-Mediums;

   (2) Entnahme der Wurzelspitzen der Adventivwurzeln von Ginseng, Zerlegen und Trennen, um einen Stammzellbereich zu erhalten;
   (3) Isolierung und Kultur: Inokulieren des Stammzellbereichs in ein Stammzellinduktionsmedium für die Dunkelkultur, um Stammzellmassen zu erhalten;
   das Stammzellinduktionsmedium umfasst 2 bis 4 mg/L Gibberellin, 0,6 bis 1 mg/L Kinetin, 2 bis 4 mg/L Indolessigsäure, 15 bis 75 mg/L Ascorbinsäure, 50 bis 150 mg/L Zitronensäure, 20 bis 60 g/L Saccharose, 1 bis 6 g/L Phytagel, 1 bis 2,4 g/L eines 1/2-MS-Mediums und 1 bis 2,5 g/L eines B5-Mediums;
   (4) Subkultur: Entnahme eines Teils der in Schritt (3) gewonnenen Stammzellmassen zur Inokulation in ein Stammzell-Subkulturmedium für die Dunkelkultur;
   das Stammzell-Subkulturmedium umfasst 2 bis 4 mg/L 2,4-Dichlorphenoxyessigsäure, 1 bis 3 mg/L Gibberellin, 0,8 bis 1,2 mg/L Kinetin, 20 bis 60 g/L Saccharose, 1 bis 6 g/L Phytagel, 1 bis 2,4 g/L eines 1/2-MS-Mediums und 0,6 bis 1,4 g/L eines WPM-Mediums; und
   (5) Flüssigkultur: Inokulieren der in Schritt (4) gewonnenen Stammzellen in ein Stammzell-Flüssigmedium für die Dunkelkultur, um Ginseng-Stammzellen zu erhalten;
   das Stammzell-Flüssigmedium umfasst 2 bis 4 mg/L 2,4-Dichlorphenoxyessigsäure, 1 bis 3 mg/L Gibberellin, 0,8 bis 1,2 mg/L Kinetin, 20 bis 60 g/L Saccharose, 1 bis 2,4 g/L eines 1/2-MS-Mediums und 0,6 bis 1,4 g/L eines WPM-Mediums.

2. Das Verfahren zur Trennung und Kultivierung von Ginseng-Stammzellen nach Anspruch 1, wobei in Schritt (3) das Stammzellinduktionsmedium 3 mg/L Gibberellin, 0,8 mg/L Kinetin, 2,5 mg/L Indolessigsäure, 50 mg/L Ascorbinsäure, 100 mg/L Zitronensäure, 40 g/L Saccharose, 3 g/L Phytagel, 1,8 g/L des 1/2-MS-Mediums und 1,5 g/L des B5-Mediums umfasst.

3. Das Verfahren zur Trennung und Kultivierung von Ginseng-Stammzellen nach Anspruch 1, wobei in Schritt (4) das Stammzell-Subkulturmedium 3 mg/L 2,4-Dichlorphenoxyessigsäure, 2 mg/L Gibberellin, 1 mg/L Kinetin, 35 g/L Saccharose, 3 g/L Phytagel, 1,8 g/L des 1/2-MS-Mediums und 0,8 g/L des WPM-Mediums umfasst.

4. Das Verfahren zur Trennung und Kultivierung von Ginseng-Stammzellen nach Anspruch 1, wobei in den Schritten (3) und (4) die Dunkelkultur in einem Bereich von 20 bis 25 °C durchgeführt wird, bis die inokulierten Stammzellen eine große Anzahl von Zellmassen bilden.

5. Das Verfahren zur Trennung und Kultivierung von Ginseng-Stammzellen nach Anspruch 1, wobei in Schritt (5) das flüssige Stammzellmedium 3 mg/L 2,4-Dichlorphenoxyessigsäure, 2 mg/L Gibberellin, 1 mg/L Kinetin, 35 g/L Saccharose, 1,8 g/L des 1/2-MS-Mediums und 0,8 g/L des WPM-Mediums umfasst.

6. Das Verfahren zur Trennung und Kultivierung von Ginseng-Stammzellen nach einem der Ansprüche 1 bis 5, wobei in Schritt (5) die Dunkelkultur in einem Bereich von 20 bis 25 °C bei 100 bis 150 U/min durchgeführt wird und die Passage alle 3 bis 5 Wochen einmal durchgeführt wird.

7. Das Verfahren zur Trennung und Kultivierung von Ginseng-Stammzellen nach einem der Ansprüche 1 bis 6, wobei in Schritt (2) die Wurzelspitzen der in Schritt (1) kultivierten Adventivwurzeln von Ginseng entnommen und unter einem Mikroskop beobachtet werden, der Stammzellbereich anhand des Meristemzellaggregationsbereichs bestimmt wird und ein Wurzelspitzen-Stammzellbereich durch Mikromanipulation mittels eines Skalpells zum Schneiden erhalten wird.

8. Das Verfahren zur Trennung und Kultivierung von Ginseng-Stammzellen nach einem der Ansprüche 1 bis 5, wobei der Ginseng aus Wildginseng, transplantiertem Wildginseng, Waldginseng und Gartenginseng ausgewählt wird.

9. Das Verfahren zur Trennung und Kultivierung von Ginseng-Stammzellen nach Anspruch 8, wobei der Ginseng Wildginseng ist.

**Revendications**

1. Un procédé pour séparer et mettre en culture des cellules souches de ginseng, comprenant :

(1) la culture des racines adventives de ginseng par un procédé en une étape, le procédé en une étape pour la culture des racines adventives de ginseng comprenant :

le lavage et la désinfection du ginseng mature, la découpe en tranches du ginseng mature désinfecté, et l'inoculation des tranches dans un milieu d'induction de racines adventives pour induire des racines adventives de ginseng, la réinoculation des racines adventives de ginseng obtenues dans un milieu d'induction de racines adventives en vue d'un repiquage et d'une propagation, et la découpe ensuite en segments des racines adventives de ginseng obtenues, et l'inoculation des segments de racines adventives dans un milieu liquide de culture afin d'obtenir des racines adventives,
le milieu d'induction de racines adventives comprend 1 à 6 mg/L d'acide naphtylacétique, 0,1 à 0,6 mg/L de kinétine, 0,2 à 1 mg/L de gibbérelline, 0,075 à 1,5 g/L d'acide citrique, 0,03 à 1 g/L d'acide ascorbique, 20 à 60 g/L de saccharose, 1 à 6 g/L de Phytagel, 1 à 4 g/L d'un milieu B5 et 1 à 2,4 g/L d'un milieu WPM,

(2) le prélèvement des apex racinaires des racines adventives de ginseng, et leur dissection et leur séparation pour obtenir une zone de cellules souches,
(3) l'isolement et la culture : l'inoculation de la zone de cellules souches dans un milieu d'induction de cellules souches en vue d'une culture à l'obscurité afin d'obtenir des masses de cellules souches,

le milieu d'induction de cellules souches comprend 2 à 4 mg/L de gibbérelline, 0,6 à 1 mg/L de kinétine, 2 à 4 mg/L d'acide indole-acétique, 15 à 75 mg/L d'acide ascorbique, 50 à 150 mg/L d'acide citrique, 20 à 60 g/L de saccharose, 1 à 6 g/L de Phytagel, 1 à 2,4 g/L d'un milieu 1/2 MS et 1 à 2,5 g/L d'un milieu B5,

(4) le repiquage : le prélèvement d'une partie des masses de cellules souches obtenues à l'étape (3) pour les inoculer dans un milieu de repiquage de cellules souches en vue d'une culture à l'obscurité,

le milieu de repiquage de cellules souches comprend 2 à 4 mg/L d'acide 2,4-dichlorophénoxyacétique, 1 à 3 mg/L de gibbérelline, 0,8 à 1,2 mg/L de kinétine, 20 à 60 g/L de saccharose, 1 à 6 g/L de Phytagel, 1 à 2,4 g/L d'un milieu 1/2 MS et 0,6 à 1,4 g/L d'un milieu WPM, et

(5) la culture liquide : l'inoculation des cellules souches obtenues à l'étape (4) dans un milieu liquide pour cellules souches en vue d'une culture à l'obscurité afin d'obtenir des cellules souches de ginseng,

le milieu liquide pour cellules souches comprend 2 à 4 mg/L d'acide 2,4-dichlorophénoxyacétique, 1 à 3 mg/L de gibbérelline, 0,8 à 1,2 mg/L de kinétine, 20 à 60 g/L de saccharose, 1 à 2,4 g/L d'un milieu 1/2 MS et 0,6 à 1,4 g/L d'un milieu WPM.

2. Le procédé pour séparer et mettre en culture des cellules souches de ginseng selon la revendication 1, dans lequel, à l'étape (3), le milieu d'induction de cellules souches comprend 3 mg/L de gibbérelline, 0,8 mg/L de kinétine, 2,5 mg/L d'acide indole-acétique, 50 mg/L d'acide ascorbique, 100 mg/L d'acide citrique, 40 g/L de saccharose, 3 g/L de Phytagel, 1,8 g/L du milieu 1/2 MS et 1,5 g/L du milieu B5.

3. Le procédé pour séparer et mettre en culture des cellules souches de ginseng selon la revendication 1, dans lequel, à l'étape (4), le milieu de repiquage de cellules souches comprend 3 mg/L d'acide 2,4-dichlorophénoxyacétique, 2 mg/L de gibbérelline, 1 mg/L de kinétine, 35 g/L de saccharose, 3 g/L de Phytagel, 1,8 g/L du milieu 1/2 MS et 0,8 g/L du milieu WPM.

4. Le procédé pour séparer et mettre en culture des cellules souches de ginseng selon la revendication 1, dans lequel, aux étapes (3) et (4), la culture à l'obscurité est réalisée dans une plage de 20 à 25 °C jusqu'à ce que les cellules souches inoculées fassent croître un grand nombre de masses cellulaires.

5. Le procédé pour séparer et mettre en culture des cellules souches de ginseng selon la revendication 1, dans lequel, à l'étape (5), le milieu liquide de cellules souches comprend 3 mg/L d'acide 2,4-dichlorophénoxyacétique, 2 mg/L de gibbérelline, 1 mg/L de kinétine, 35 g/L de saccharose, 1,8 g/L du milieu 1/2 MS et 0,8 g/L du milieu WPM.

6. Le procédé pour séparer et mettre en culture des cellules souches de ginseng selon l'une quelconque des revendications 1 à 5, dans lequel, à l'étape (5), la culture à l'obscurité est réalisée dans une plage de 20 à 25 °C à 100-150 t/min, et le passage a été effectué à raison d'une fois toutes les 3 à 5 semaines.

7. Le procédé pour séparer et mettre en culture des cellules souches de ginseng selon l'une quelconque des revendications 1 à 6, dans lequel, à l'étape (2), les apex racinaires des racines adventives de ginseng cultivées à l'étape (1) sont prélevées et observées au microscope, la zone de cellules souches est déterminée sur la base de la zone d'agrégation des cellules méristématiques, et une zone de cellules souches d'apex racinaires est obtenue par micromanipulation en utilisant un scalpel pour la découpe.

8. Le procédé pour séparer et mettre en culture des cellules souches de ginseng selon l'une quelconque des revendications 1 à 5, dans lequel le ginseng est choisi parmi du ginseng sauvage, du ginseng sauvage transplanté, du ginseng de forêt et du ginseng de jardin.

9. Le procédé pour séparer et mettre en culture des cellules souches de ginseng selon la revendication 8, dans lequel le ginseng est du ginseng sauvage.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104472359 A

- EP 2962552 A1

**Non-patent literature cited in the description**

- **EUN-KYONG LEE et al.** *Nature Biotechnology*, 2010, vol. 28, 1213-1217
- **BAOBAO QIANG et al.** *Chem. Biodiversity*, 2020, 17

- **SIVAKUMAR G et al.** *Eng. Life Sci.*, 2005, vol. 5, 333-342